# EUROPEAN PATENT APPLICATION

(11) **EP 3 656 850 A1**
(43) Date of publication of application: **27.05.2020**
(21) Application number: 18756144.4
(22) Date of filing: 17.07.2018
(51) Int. Cl.: C12N 5/0775, A61K 8/9789, A61K 8/9794, A61K 36/28, A61K 36/63, A61K 36/88, A61P 17/00, A61P 43/00, A61Q 19/08, A61K 127/00

(54) **MESENCHYMAL-STEM-CELL INDUCTION AGENT**

(30) Priority: 18.07.2017 JP 2017139264
(71) Applicant: Shiseido Co., Ltd., Tokyo 104-0061 (JP)
(72) Inventor: EZURE, Tomonobu, Yokohama-shi Kanagawa 224-8558 (JP)
(74) Representative: Santarelli
(86) International application number: PCT/JP2018/026778
(87) International publication number: WO 2019/017355

(57) **Abstract**

[PROBLEM]

To provide a mesenchymal stem cell attracting agent, as well as an aging-factor production inhibiting agent, anti-aging agent, and dermal regeneration promoting agent including the attracting agent.

[SOLVING MEANS]

It was found that iris extract, olive leaf extract, and chamomile extract have a mesenchymal stem cell inducing action, which led to the invention of a mesenchymal stem cell attracting agent. Furthermore, it was discovered that mesenchymal stem cells inhibit aging factor production and activate dermal fibroblasts, whereby an aging-factor production inhibiting agent, anti-aging agent, and dermal regeneration promoting agent including the attracting agent were invented.

## Description

### FIELD

The present invention relates to a mesenchymal stem cell attracting agent including at least one selected from the group consisting of iris extract, olive leaf extract, and chamomile extract and an aging-factor production inhibiting agent, anti-aging agent, and dermal regeneration promoting agent including the attracting agent.

### BACKGROUND

In the field of regeneration medicine, which aims to newly regenerate tissue, it has been attempted that various stem cells is used to restore the function of lost tissue. Regenerative medicine has been applied not only to the medical field but also to cosmetic field relating to hair and skin regeneration.

Stem cells are defined as cells having a self-replication ability and the differentiation potential to differentiate into other kinds of cells. In addition to stem cells having pluripotency to differentiate into all of the cell types which form an individual body such as embryonic stem cells (ES cells) and induced pluripotent stem cells (iPS cells), somatic stem cells having multipotency, but not pluripotency, to differentiate into multiple types of cells are known as stem cells to exist in the living body of an adult.

It is difficult to use embryonic stem cells in research and clinical uses, and is further difficult to use in the cosmetic field, since embryonic stem cells can only be obtained from embryos in the early stages of development, which causes the maternal risks at the time of collection and ethical concerns. Furthermore, regarding induced pluripotent stem cells, though there are few ethical concerns, there is a risk of cancer brought about by the introduction of genes. Thus, in the field of cosmetics, research on the use of somatic stem cells is advancing more than the use of embryonic stem cells and induced pluripotent stem cells. The frequency of occurrence of somatic stem cells is very low, and isolation thereof is difficult. Furthermore, it is also difficult to cultivate somatic stem cells in large quantities while maintaining the self-replication ability and differentiation ability.

Somatic stem cells are known to contribute to tissue repair at the time of tissue damage, maintenance of tissue homeostasis, and aging. Somatic stem cells are known to be present in various tissues. As the stem cells present in the skin tissue, epidermal stem cells present in the epidermal layer and mesenchymal stem cells present in the dermal layer and subcutaneous fat layer are known.

It is well known that epidermal stem cells are present in the epidermal basal layer in the epidermis. It has also been reported that hair follicle epithelial stem cells and skin pigment stem cells are present in the area called the hair follicle bulge area. It has been reported that the dermis contains skin-derived precursor cells (SKP) that differentiate into multiple cell lines such as fat, glial cells, cartilage, and muscle (Non-Patent Literature 1: Wong CE et al., J Cell Biol. 175: 1005-1015, 2006), and that cells referred to as dermal mesenchymal stem cells have been isolated (Patent Literature 1: WO 2001/034106).

Mesenchymal stem cells are known to be attracted to trauma sites when the skin is damaged. Mesenchymal stem cells attracted to the trauma are thought to activate the surrounding skin cells so as to promote cell proliferation, and themselves differentiate into skin cells so as to block trauma thereby contribute to healing. It has been shown that certain flavonoids have a mesenchymal stem cell attracting action (Non-Patent Literature 2: PLOS ONE DOI: 10.137/journal.pone.0144166). In addition to bone marrow-derived mesenchymal stems cells that are transported via blood vessels, adipose stem cells present in the subcutaneous tissue, and dermal mesenchymal stem cells present around organs such as the sebaceous glands, sweat glands, and hair follicles and near blood vessels are considered to be mesenchymal stem cells involved in the healing of skin damage. To date, mainly bone marrow-derived stem cells have been studied as mesenchymal stem cells. However, after adipose stem cells are isolated from adipose tissue, adipose stem cells come to be expected for application to various regenerative treatments due to the ease of availability and multipotency to differentiate into several cell types (Non-Patent Literature 3: World J Stem Cells 2014, January 26: 6(1), 65-68). Although dermal mesenchymal stem cells are present near blood vessels, there are many unknown points regarding the differentiation ability and properties thereof. Thus, future research is expected for the application thereof.

### [CITATION LIST]

### [PATENT LITERATURE]

[PTL 1] WO 2011/034106
[PTL 2] Japanese Unexamined Patent Publication (Kohyo) No. 2013-507956

### [NON-PATENT LITERATURE]

[NPL 1] J Cell Biol. 175: 1005-1015, 2006
[NPL 2] PLOS ONE DOI: 10.137/journal.pone.0144166
[NPL 3] World J Stem Cells 2014 January 26; 6(1): 65-68
[NPL 4] Cell, (2011) 146, 761-771
[NPL 5] Journal of Dermatological Science (2007) 48, 15-24
[NPL 6] Stem Cell Research & Therapy 2014, 5; 21

### SUMMARY

### [TECHNICAL PROBLEM]

The present invention aims to develop an agent having a stem cell attracting action.

### [SOLUTION TO PROBLEM]

While developing cosmetics that improve skin aging phenomena, the present inventors screened cosmetic materials capable of attracting mesenchymal stem cells and have discovered that iris extract, olive leaf extract, and chamomile extract are able to attract mesenchymal stem cells, thereby they have invented to the present invention. Thus, the present invention relates to the following.
[1] A mesenchymal stem cell attracting agent, comprising at least one selected from the group consisting of iris extract, olive leaf extract, and chamomile extract.
[2] An aging-factor production inhibiting agent comprising the attracting agent according to item 1.
[3] An anti-aging agent comprising the attracting agent according to item 1 or the aging-factor production inhibiting agent according to item 2.
[4] A dermal regeneration promoting agent comprising the attracting agent according to item 1.
[5] At least one extract selected from the group consisting of iris extract, olive leaf extract, and chamomile extract for use in treating a diseases which is treated or improved by mesenchymal stem cells through attracting mesenchymal stem cells.
[6] The extract according to item 5, wherein said disease is articular cartilage disease, such as osteoarthritis, cardiac disease, dystrophy, dermal wound or damage.
[7] A cosmetic method or non-therapeutic method through attracting mesenchymal stem cells present in the dermis, comprising the administration of at least one selected from the group consisting of iris extract, olive leaf extract, and chamomile extract.
[8] The cosmetic or non-therapeutic method according to item 7, wherein aging factor production is suppressed by attraction of mesenchymal stem cells.
[9] The cosmetic or non-therapeutic method according to item 7, wherein dermal regeneration is prompted by attraction of mesenchymal stem cells.
[10] The cosmetic or non-therapeutic method according to any one of items 7 - 9, wherein aging in terms of external appearance, such as sagging or wrinkle, is improved by attraction of mesenchymal stem cells.
[11] A mesenchymal stem cell attracting agent for use in the treatment or prevention of aging.
[12] A mesenchymal stem cell attracting agent for use in a dermal regenerative treatment.
[13] The attracting agent according to item 11 or 12, wherein the attracting agent comprises at least one extract selected from the group consisting of iris extract, olive leaf extract, and chamomile extract.
[14] Non-therapeutic use of at least one extract selected from the group consisting of iris extract, olive leaf extract, and chamomile extract as a mesenchymal stem cell attracting agent.
[15] Non-therapeutic use of at least one extract selected from the group consisting of iris extract, olive leaf extract, and chamomile extract for improving aging in terms of external appearance, such as sagging or wrinkle through attraction of mesenchymal stem cells.
[16] A method for the inhibition of aging, comprising the administration of at least one selected from the group consisting of iris extract, olive leaf extract, and chamomile extra to a subject requiring the attraction of mesenchymal stem cells.
[17] A method for the promotion of the regeneration of the dermis, comprising the administration of at least one selected from the group consisting of iris extract, olive leaf extract, and chamomile extract to a subject in need of dermal regeneration.
[18] Use of at least one extract selected from the group consisting of iris extract, olive leaf extract, and chamomile extract for preparing a formulations for the treatment or control of aging by attracting mesenchymal stem cells present in the dermis.
[19] Use of at least one extract selected from the group consisting of iris extract, olive leaf extract, and chamomile extract for preparing a dermal regenerative agent by attracting mesenchymal stem cells present in the dermis.

### [ADVANTAGEOUS EFFECTS OF INVENTION]

The mesenchymal stem cell attracting agent of the present invention has a mesenchymal stem cell attracting action. Mesenchymal stem cells can activate dermal fibroblasts via the action of at least one of differentiation into dermal fibroblasts, the production of cytokines, and the suppression of aging factors, whereby it is possible to suppress or improve aging and to regenerate the dermis.

### BRIEF DESCRIPTION OF DRAWINGS

[FIG. 1] FIG. 1(A) shows a schematic diagram of a Transwell system in which normal (young) fibroblasts are cultured in an upper layer and senescent fibroblasts are cultured in a lower layer.
FIGS. 1(B) through 1(D) are graphs showing a comparison of (B) collagen 1A1 (COL1A1), (C) elastin (ELN), and (D) matrix metalloprotease 1 (MMP1) gene expression levels between a case in which cells are not cultured in the lower layer (Blank) and a case in which senescent fibroblasts are cultured in the lower layer (+Senescent).
[FIG. 2] FIG. 2 is a graph showing the change in MMP1 gene expression in the upper layer fibroblasts (B) when the CFD gene expression of the senescent fibroblasts of the lower layer of the Transwell system is suppressed (A).
[FIG. 3] FIG. 3 is a graph showing a comparison in the gene expression levels of the aging factors (A) IGFBP7, (B) RSPO4, and (C) CFD between senescent fibroblasts and normal (young) fibroblasts.
[FIG. 4] FIG. 4 is a graph showing that when IGFBP7 and RSPO4 were added, (A) the expression of the MMP1 gene increased in a dose-dependent manner, and (B) the expression of the collagen 1 gene decreased.
[FIG. 5] FIG. 5 is a photograph (A) and graph (B) showing that due to the presence of mesenchymal stem cells in a co-culture system of fibroblasts and mesenchymal stem cells, the thickness of the fibroblasts layer increased.
[FIG. 6] FIG. 6 is a photograph (A) and graph (B) showing that due to the presence of mesenchymal stem cells in a co-culture system of collagen gel and mesenchymal stem cells, the size of the collagen gel decreased.
[FIG. 7] FIG. 7 is a graph showing that due to the presence of mesenchymal stem cells in a co-culture system of senescent fibroblasts and mesenchymal stem cells, the expression of IGFBP7, which is an aging factor, decreased.

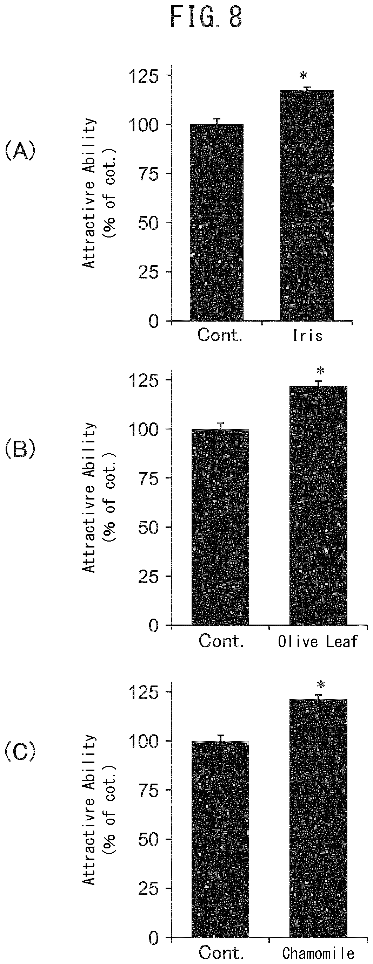

[FIG. 8] FIG. 8 is a graph showing that (A) iris extract, (B) olive leaf extract, and (C) chamomile extract each have a mesenchymal stem cell attracting action.

### DESCRIPTION OF EMBODIMENTS

The present invention is directed to a mesenchymal stem cell attracting agent comprising at least one selected from the group consisting of iris extract, olive leaf extract, and chamomile extract. Mesenchymal stem cell attracting agent refers to an agent which can attract mesenchymal stem cells.

Iris extract refers to an extract of a perennial plant from the *Iridaceae* family, which is native to the Mediterranean coast. More preferably, iris extract refers to an extract of, from the *Iridaceae* family, *Fragaria nipponica, Iris florentina, Iris pallida,* orris (*Iris germanica Var. florentina*) or species related thereto. The portions of the plant from which the extract can be obtained include the stalk, leaves, flowers, or roots. When roots are used as the raw material, fragrant extracts can be obtained. Thus, root extracts are preferred. Any method can be used as the extraction method therefor. For example, extract can be obtained by performing steam distillation or solvent extraction on dried roots. Furthermore, as the solvent, any extraction solvent can be used. An extract obtained by extraction with an ethanol aqueous solution or 50% (v/v) butylene glycol can be used.

Olive leaf extract is an extract derived from the leaves of the olive tree (*Olea europaea*), which is a member of the family *Oleacaea*, which is native to the Mediterranean coast from the Punjab region of India, or species related thereto. Olive leaf extract has an antioxidant action, a collagen production promoting action, an anti-inflammatory action, and an antiallergenic action. Olive leaf extract is preferably extracted from the leaves of olive trees as a raw material. Any method can be used as the extraction method, for example, steam distillation or solvent extraction. Furthermore, as the solvent, any extraction solvent can be used. An extract obtained by extraction with 50% (v/v) butylene glycol can be used.

Chamomile extract refers to an extract from chamomile (*Matricaria chamomilla*), which is a member of the annual *Asteraceae* family and which is widely distributed from Europe to Western Asia, or a species related thereto. Chamomile extract is preferably extracted from the flower of chamomile as a raw material. Any method can be used as the extraction method, for example, steam distillation or solvent extraction. Furthermore, as the solvent, any extraction solvent can be used. An extract obtained by extraction with a 50% (v/v) ethanol aqueous solution or butylene glycol can be used.

Since these extracts are cosmetic materials, it is preferable to incorporate the extracts in a cosmetic. One extract may be used alone, or two or more extract can be used by mixture as a mesenchymal stem cell attracting agent. The mesenchymal stem cell attracting agent may be used in combination with an existing mesenchymal stem cell attracting agent, such as SDF1α (CXCL20) or the like. A composition comprising iris extract, olive leaf extract, and/or chamomile extract can be used as a mesenchymal stem cell attracting agent.

As the extraction solvent used to obtain the plant extract, water, or an organic solvent, for example, an aliphatic monohydric alcohol such as methanol, ethanol, or propanol, an aliphatic polyhydric alcohol such as propylene glycol or 1-3-butylene glycol, a ketone such as acetone, an ester such as diethyl ether, dioxane, acetonitrile, or ethyl acetate, an aromatic compound such as xylene, benzene, or toluene, or an alkyl halide such as chloroform may be used. These extraction solvents may be used alone or in a combination of two or more thereof. The mixing ratio of the mixed extraction solvents is not particularly limited and can be appropriately adjusted. As the extraction solvent, a water-containing extraction solvent containing at least water is preferable.

The extraction method for obtaining the plant extract is not particularly limited, and a conventionally known general extraction method can be used. During extraction, the extract portion of each plant can be used as is or dried. After extraction, if necessary, a purification treatment such as filtration, deodorization, bleaching or the like can be appropriately performed. An adipose stem cell attracting agent and an anti-sag or anti-aging agent can be added to the final product. The concentration of each extract contained in the final product is not particularly limited. For example, from the viewpoint of bringing about a sufficient adipose stem cell attracting effect, the extracts are typically blended in a range of 0.001 to 10% by weight, preferably in the range of 0.001 to 1% by weight, most preferably in the range of 0.01 to 0.5% by weight.

In the present invention, "mesenchymal stem cells" refers to stem cells derived from any tissue generally classified as mesenchymal. A tissue classified as mesenchymal include bone marrow, placenta, adipose tissue, and dermal tissue. As examples of stem cells derived from these tissues, bone marrow-derived mesenchymal stem cells (hereinafter sometimes referred to simply as hematopoietic stem cells), placental-derived mesenchymal stem cells, adipose tissue-derived mesenchymal stem cells (hereinafter sometimes referred to simply as adipose stem cells), dental pulp mesenchymal stem cells, cord blood-derived mesenchymal stem cells, and dermal-derived mesenchymal stem cells may be used. Furthermore, some dermal papilla cells are considered to be mesenchymal stem cells. The mesenchymal stem cell attracting agent of the present invention may have an action to attract mesenchymal stem cells derived from any tissue. For example, in view of the application in the fields of cosmetics and skin regeneration, it is preferable to have an attracting action on dermal stem cells, adipose stem cells, and hair dermal papilla cells, particularly from the viewpoint of exhibiting an attracting action by transdermal administration.

Bone marrow-derived mesenchymal stem cells are stem cells present in bone marrow. Bone marrow stem cells have the potency to differentiate into various tissues such as myocardial cells, skeletal muscle cells, vascular endothelial cells, cartilage cells, bone cells, and fat cells. Bone marrow-derived mesenchymal stem cells can be characterized by CD105, CD73, CD90 positive markers, but the bone marrow-derived mesenchymal stem cells are not intended to be limited to cells expressing these markers. Bone marrow mesenchymal stem cells are thought to be released into the blood in response to signals such as tissue damage and are thought to perform a healing function in the area of the wound.

Adipose tissue-derived mesenchymal stem cells can also be simply referred to as adipose stem cells and are a type of mesenchymal stem cell present in adipose tissue. Adipose stem cells are known to have a migratory ability (Non-Patent Literature 3 and Patent Literature 2) and are thought to be able to migrate in adipose tissue. The adipose stem cells are characterized by the expression of the CD90 and CD105 markers. However, adipose stem cells are not intended to be limited to cells expressing these markers.

Dermal-derived mesenchymal stem cells can also be simply referred to as dermal stem cells and are stem cells present in the dermis. Dermal stem cells contribute to the homeostatic maintenance of the dermal layer by supplementing fibroblasts through differentiation into fibroblasts or the activation of fibroblasts. Dermal mesenchymal stem cells are mainly present around the microvascular sites and around organs such as the sebaceous glands, sweat glands, hair follicles and the like, as a pericyte-like cell population in the dermal layer. Dermal mesenchymal stem cells are considered to be activated upon skin damage, differentiated into fibroblasts or muscle fibroblasts, and contribute to skin regeneration and repair. Dermal stem cells are characterized by double positive expression of CD34 and NG2. However, dermal mesenchymal stem cells are not intended to be limited to cells expressing these markers. Dermal stem cells are thought to be supplemented through blood vessels, since capillary vessels are stretched around each organ in which dermal stem cells are present. Thus, dermal stem cells may have properties similar to bone marrow mesenchymal stem cells.

The attraction of mesenchymal stem cells can be confirmed both *in vitro* and *in vivo.* In *in vitro* experiments, the attracting effect of the mesenchymal stem cell attracting agent can be determined by culturing mesenchymal stem cells in a medium having a concentration difference of mesenchymal stem cell attracting agent and detecting migration. For confirmation *in vivo*, the presence or absence of attractive action can be examined by calculating the number or proportion of mesenchymal stem cells in a section of tissue after administration of the mesenchymal stem cell attracting agent.

The mesenchymal stem cell attracting agent of the present application, when administered, can attract mesenchymal stem cells to the administration site. The cells that can be attracted are not limited as long as they are mesenchymal stem cells, but in particular bone mallow stem cells, dermal stem cells, hair papilla cells, and adipose stem cells can be attracted. Further, in a more preferable embodiment, bone mallow stem cells and dermal stem cells can be attracted. In the case of percutaneous administration, mesenchymal stem cells are attracted to the dermal layer. The attracted mesenchymal stem cells suppress aging of the dermis through various actions and contribute to regeneration of the dermis. Examples of such actions include the suppression of aging factors secreted by aging cells, the activation of normal cells, and differentiation into dermal cells.

In cells, the ability to divide decreases as time elapses, and the cell activity also changes. Such cells are called senescent cells, and senescent cells can be affected not only by elapsed time but also by stresses such as ultraviolet rays and active oxygen. Senescent cells decrease the secretion of substances secreted by normal cells and secrete substances called aging factors. The inventors have discovered that IGFBP7, CFD, and RSPO 4 are aging factors. Aging factors are factors produced from aged cells and contribute to aging by acting on other cells. By acting on fibroblasts, the aging factors specified in the present invention are involved in skin aging via the decreased production of dermal components such as collagenous fibers, e.g., collagen, and elastic fibers, e.g., elastin and fibrin, and the promotion of collagenous fiber or elastic fiber degrading-enzymes such as collagenase or protease. Mesenchymal stem cells can suppress the production of aging factors by aging cells and suppress the function of the aging factors. Mesenchymal stem cells attracted to dermis inhibit the production and function of aging factors and can suppress the reduction in and decomposition of the dermal components, whereby the aging of the dermis can be suppressed. Thus, the mesenchymal stem cell attracting agent of the present invention can be used as aging-factor production inhibiting agent or as an anti-aging agent for the skin.

It is also known that mesenchymal stem cells increase the activity of dermal layer cells. The mechanism of action thereof is uncertain and is not limited to the following, but it is considered that the action is brought about by cytokines such as TGF-β produced by mesenchymal stem cells. In this way, mesenchymal stem cells promote the proliferation of dermal fibroblasts and promote the production of elastic components of the dermis such as collagenous fibers and elastic fibers, and are also involved in the maturation of these elastic fibers and collagenous fibers. In addition to promoting the division of dermal fibroblasts, mesenchymal stem cells themselves differentiate into fibroblasts to supply dermal cells, thereby regenerating the dermis. The mesenchymal stem cell attracting agent of the present invention can be used as a collagenous fiber or elastic fiber (e.g., collagen, elastin or fibrin) production promoting agent, a dermal regenerating agent, or an anti-aging agent.

Dermal regeneration primarily means that the skin elasticity is healed through increased and maturated elastic components in the dermis. Wrinkles and sagging, which deteriorate the appearance of the skin and impart an aged impression, are attributed to a decrease in the content of elastic fibers and collagenous fibers, which are the elastic components in the dermal layer. Thus, the mesenchymal stem cell attracting agent of the present invention can be used as a wrinkle-improving agent, an agent for improving sag and toughness, and an anti-aging agent.

The dermis is primarily composed of cell components such as fibroblasts, histiocytes, mast cells, plasma cells, and dendric cells, as well as interstitial components filling between the cells. Interstitial components are roughly divided into collagenous fibers composed of collagen, elastic fibers composed of fibrin and elastin, and substrates mainly composed of glycoproteins and proteoglycans present between fibers and cells.

The mesenchymal stem cell attracting agent of the present invention can be administered to a subject suffering from aging in appearance, such as sagging or wrinkle. The causes of sag include a decrease in the function of the facial muscles, a decrease in the elasticity of the skin supporting the facial muscles, an increase in subcutaneous fat, etc. These causes cause sagging independently or in cooperation to result in aging in appearance. In the present invention, the mesenchymal stem cell attracting agent is administered to a subject suffering from sagging of the skin, but in such subjects, it is preferable that the main cause of sag be a reduction in the elasticity of the skin. In another embodiment, a mesenchymal stem cell attracting agent can also be administered to a subject who requires regeneration of dermis. For such a subject, a mesenchymal stem cell attracting agent can also be administered to the damaged area of the skin. Skin injuries include trauma, wounds, burns, and inflammation. In particular, the therapeutic effects of mesenchymal stem cells on dermatitis, such as atopic dermatitis, are known. Thus, the mesenchymal stem cell attracting agent can be used as a remedy for skin damage and dermatitis, in particular atopic dermatitis.

It is known that mesenchymal stem cells differentiate into various cells such as fat cells, osteoblasts, cartilage cells, muscle cells, nerve cells, etc., in addition to dermal fibroblasts (Non-Patent Literature 3). By utilizing such potency to differentiate into various cells, the mesenchymal stem cell attracting agent of the present invention can be used for the treatment of various diseases. Thus, the mesenchymal stem cell attracting agent of the present invention, which contains at least one selected from the group consisting of iris extract, olive leaf extract, and chamomile extract, can be used as a pharmaceutical composition for the treatment or prevention of such diseases. Such therapeutic/prophylactic uses are not limited to uses on the skin, such as dermal regeneration, aging improvement, wound healing or the like, as described above. For example, Patent Literature 3 discloses that cartilage cells derived from adipose stem cells, which are mesenchymal stem cells, can be used for the treatment of articular cartilage diseases such as osteoarthritis, osteoblasts derived from adipose stem cells can be applied to bone defect sites, and that mesenchymal stem cells contribute to an improvement in cardiac function by differentiation into myocardium and blood vessel cells. Further, factors secreted from adipose stem cells result in enhanced angiogenesis, reduced cell apoptosis, and promotion of neural germination. Thus, in addition to applications in direct regenerative medicine, in which adipose stem cells differentiate into target cells, applications in which indirect regenerative medicine via factor secretion is expected. Therefore, the mesenchymal stem cell attracting agent of the present invention can be used for treating or ameliorating the therapeutic target symptoms of the mesenchymal stem cells. Mesenchymal stem cells can bring about an improvement in target symptoms including hair growth promotion, wound healing, skin regeneration, muscle regeneration, soft tissue regeneration, bone regeneration, myocardial regeneration, and nerve regeneration. Among these, regarding the relationship between adipose stem cells, which are mesenchymal stem cells, and hair growth promotion, Non-Patent Literature 4 discloses that mesenchymal stem cells contribute to the regeneration of hair follicles by functioning as skin Niche cells which control the activity of skin stem cells, and that it is possible to promote hair growth by attracting adipose stem cells. Regarding the relationship between adipose stem cells and wound healing and/or skin regeneration, Non-Patent Literature 5 discloses that contact between adipose stem cells and dermal fibroblasts promotes the proliferation of the dermal fibroblasts. Thus, it is known that mesenchymal stem cells can be used for wound healing and skin regeneration. Further, regarding the relationship between adipose stem cells and muscle regeneration, Non-Patent Literature 6 discloses the therapeutic effect of an adipose stem cell implantation test on congenital muscular dystrophy targeting collagen type 6, and discloses that adipose stem cells, which are mesenchymal stem cells, can also be used for muscle regeneration. The disease to be treated or improved by mesenchymal stem cells includes articular cartilage diseases such as osteoarthritis, heart disease, muscular dystrophy, or aging, damage or wound in tissue. The tissue to be treated includes in particular tissues differentiated from mesenchymal stem cells, for example dermis, blood vessel, muscle, bone, hair, nerves, and therefore the mesenchymal attracting agent is used for regenerating these tissues. Thus, the composition of the present invention can be used as a therapeutic or prophylactic composition for articular cartilage diseases such as osteoarthritis, heart disease, muscular dystrophy, or regeneration therapy of various organs such as the heart, liver, bones, muscles, nerves, skin, or hair.

The mesenchymal stem cell attracting agent of the present invention can be administered by any means such as oral or parenteral administration, but percutaneous administration is preferred from the viewpoint of directly administering to the skin. The percutaneous administered mesenchymal stem cell attracting agent can penetrate through accessory organs such as the pores and sweat glands present in the skin. The accessory organs reach the deepest parts of the normal dermal layer. Thus, the drug can be delivered to the deepest parts of the dermal layer by penetrating through the accessory organs. The viscosity, surface tension, hydrophilicity and hydrophobicity of the skin anti-sag or anti-aging agent can appropriately selected. Further, high frequency vibrations or sound waves can also be applied after application of the skin anti-sag or anti-aging agent of the present invention to promote penetration into the deep dermis. From the viewpoint of administering to the damaged parts of the skin, the agent can be used for percutaneous administration if formulated as an ointment or poultice.

The mesenchymal stem cell attracting agent of the present invention can be incorporated in cosmetics, pharmaceuticals, or quasi-drugs. For cosmetic purposes, the mesenchymal stem cell attracting agent is preferably incorporated in a cosmetic. The form of the cosmetic to be formulated is arbitrary and can be blended in the form of a liquid, gel, foam, emulsion, cream, ointment, sheet, etc. The cosmetic, pharmaceutical or quasi-drug to be formulated, in which the mesenchymal stem cell attracting agent can be used, includes a basic cosmetic such as an external medicinal preparation, facial lotion, emulsion, cream, ointment, lotion, oil, pack, a makeup cosmetic such as a facial wash, skin cleanser, depilatory cream, hair remover, aftershave lotion, pre-shave lotion, shaving cream, foundation, lipstick, blush, eye shadow, eyeliner, or mascara, or various products such as a shampoo, rinse, hair treatment, hair pre-treatment, hair conditioner, perming agent, hair tonic, a hair care product such as a hair dye or pilatory/hair growth product, or a bath additive.

The mesenchymal stem cell attracting agent of the present application can be administered to a human by blending into a cosmetic, medicament or the like. The administration form is not limited thereto. For example, the mesenchymal stem cell attracting agent of the present application can be for the isolation of mesenchymal stem cells *in vitro,* using the mesenchymal stem cell attracting property.

All references mentioned in the present specification are incorporated by reference in their entirety.

The embodiments of the present invention described below are for illustrative purposes only and do not limit the technical scope of the present invention. The technical scope of the present invention is limited only by the description of the claims. Modifications of the present invention, for example, the addition, deletion or substitution of the constituent features of the present invention, can be carried out on condition that the essence of the present invention be maintained.

### EXAMPLES

### Example 1: Effects of senescent cells on normal cells

### Cell Culture

Dermal fibroblasts collected from the back skin of an adult (21 years old) were cultured in Dulbecco's Modified Eagle Medium supplemented with 10% FBS. As normal fibroblasts (young fibroblasts), cells with a culture period of less than 1 month and a Pdl < 30 were used. The same cells were cultured for 3 months or more and slowly proliferating cells (having a doubling time of less than 0.5/week and a Pdl > 50) were used as the senescent fibroblasts. Normal fibroblasts were seeded in the upper layer of a six-well Transwell system (Falcon; Franklin Lakes, NJ) filled with Dulbecco's Modified Eagle Medium supplemented with 10% FBS to a density of 1250 cells/cm². Sub-constructed senescent fibroblasts and normal fibroblasts were each seeded in two wells of the lower layer of the Transwell system. After combining the upper layer and the lower layer of the Transwell system for 2 days under a humidified atmosphere at 37 °C and 5% CO₂, the cells in the upper layer and the cells in the lower layer were recovered using Qiazole (Qiagen).

### Quantitative RT-PCR

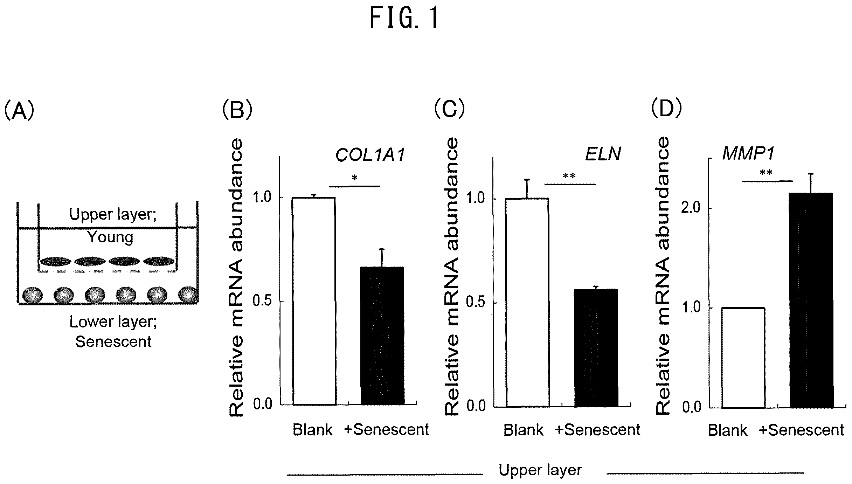

From the recovered cell lysate, RNA was extracted using an RNeasy Protect Kit (Qiagen) according to the product description and translated into cDNA using Superscript VILO (Invitrogen) according to the product description. Real time PCR was performed on LightCycler (Roche) using 28S rRNA as an internal standard and using primer pairs for the following genes. The results are shown in FIG. 1. The cycle threshold (Ct value) calculated using Lightcycler software ver. 3.5 was normalized for each sample GAPDH mRNA.

**[Table 1]**

| | | |
|---|---|---|
| *MMP1* forward | ATTTGCCGACAGAGATGAAGTCC | (SEQ ID No.1) |
| *MMP1* reverse | GGGTATCGGTGTAGCACATTCTG | (SEQ ID No. 2) |
| *ELN* forward | TGTCCATCCTCCACCCCTCT | (SEQ ID No. 3) |
| *ELN* reverse | CCAGGAACTCCACCAGGAAT | (SEQ ID No.4). |
| *COL1A1* forward | AGCAGGCAAACCTGGTGAAC | (SEQ ID No. 5) |
| *COL1A1* reverse | AACCTCTCTCGCCTCTTGCT | (SEQ ID No. 6) |
| *GAPDH* forward | GAAGGTGAAGGTCGGAGT | (SEQ ID No. 7) |
| *GAPDH* reverse | GAAGATGGTGATGGGATTTC | (SEQ ID No. 8) |

As shown in FIG. 1(A), when senescent fibroblasts were seeded into the lower layer, the expression of MMP1 was significantly increased as compared with the case where cells were not seeded in the lower layer, and the expressions of elastin and collagen were significantly decreased. When normal fibroblasts (young fibroblasts) were seeded in the lower layer, no significant change was shown as compared with the case where cells were not seeded in the lower layer. From these results, it was revealed that a change in gene expression occurred in dermal fibroblasts which shared the medium through the secretion of some aging factor from the senescent cells.

### Identification of genes having increased expression in senescent fibroblasts

The differences in gene expression between young fibroblasts and senescent fibroblasts was examined by microarray analysis. Genes that showed an expression level not less than 2.5 times the expression of the gene expression of young fibroblasts were identified. The genes listed in the table below were candidates for aging factors:

**[Table 2]**

| Gene Name | Description | Systematic Name | Fold Increase |
|---|---|---|---|
| RSP04 | Homo sapiens R-spondin family, member 4 | NM_001029871 | 5.9 |
| IGFBP7 | Homo sapiens insulin-like growth factor binding protein 7 | NM_001553 | 3.6 |
| CFD | Homo sapiens complement factor D | NM_001928 | 2.7 |

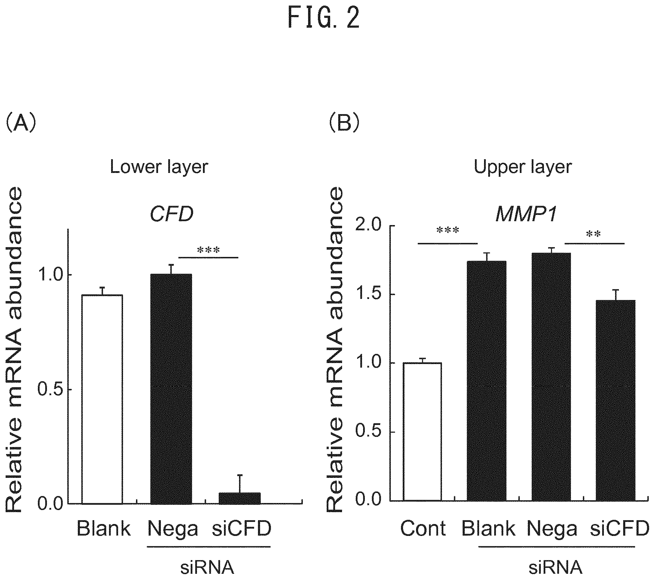

An siRNA was designed to knock down CFD from among these aging factor candidate genes (SI00030100 or SI00030107, Qiagen; both have a similar inhibitory effect) and siRNA (SI030640318; Qiagen) was used as a negative control. The siRNA was introduced into senescent fibroblasts and cells in which the CFD gene was knocked down and the cells were seeded in the lower layer of the Transwell system in accordance with the above cell culture method and cultured for 2 days with the cells of the upper layer. The cells of the lower layer and the cells of the upper layer were recovered. For the cells of the lower layer, the CFD expression was examined, and for the cells of the upper layer, the MMP1 gene expression was examined. The expression of MMP1 in cells of upper layer decreased when CDF knocked down senescent fibroblasts were seeded in the lower layer. The results are shown in FIG. 2. From these results, CFD was identified as an aging factor.

### Aging factor expression in senescent cells

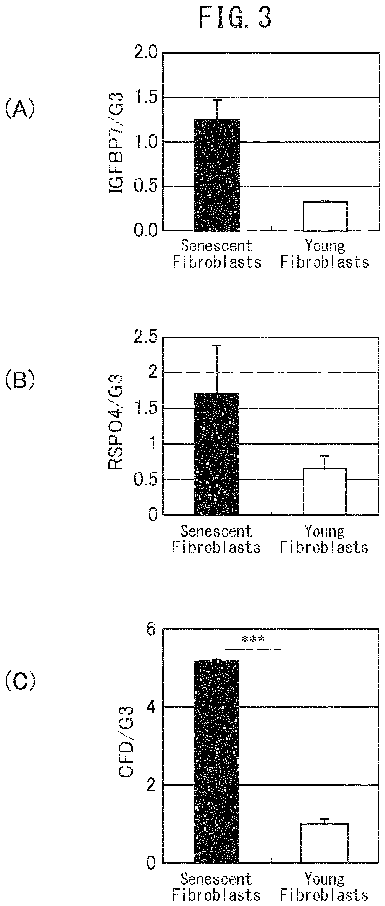

The expressions of the aging factor (CFD) and candidate aging factors (IGFBP 7, RSPO 4) specified by the present inventors were compared between senescent fibroblasts and young fibroblasts. Senescent fibroblasts and young fibroblasts were each cultured and recovered using Qiazole (Qiagen). The collected cell lysate was examined for the expression of aging factors using the same method as RT-PCR described above. The following primer pairs were used. The results are shown in FIG. 3. The aging factors and candidate aging factors specified in the present invention were both present in the senescent fibroblasts, indicating that the expression was high.

**[Table 3]**

| | | |
|---|---|---|
| CFD forward | CATCTGGTTGGTCTTTATTGAGC | (SEQ ID No. 9) |
| CFD reverse | CATGCTGATCTCGAACTCCTG | (SEQ ID No. 1 0) |
| IGFBP7 forward | TCAAAGGACAGAACTCCTGCCTGGTGA | (SEQ ID No. 1 1) |
| IGFBP7 reverse | GCTGAAGCCTGTCCTTGGGAATTGGATG | (SEQ ID No. 12) |
| RSP04 forward | CCAGCAGAGGCTCTTCCTGTTCATCC | (SEQ ID No. 13) |
| RSP04 reverse | TCCTGGCTGAAGCAGCTCTCACAAG | (SEQ ID No. 14) |
| *GAPDH* forward | GAAGGTGAAGGTCGGAGT | (SEQ ID No. 7) |
| *GAPDH* reverse | GAAGATGGTGATGGGATTTC | (SEQ ID No. 8) |

### Addition of aging factors

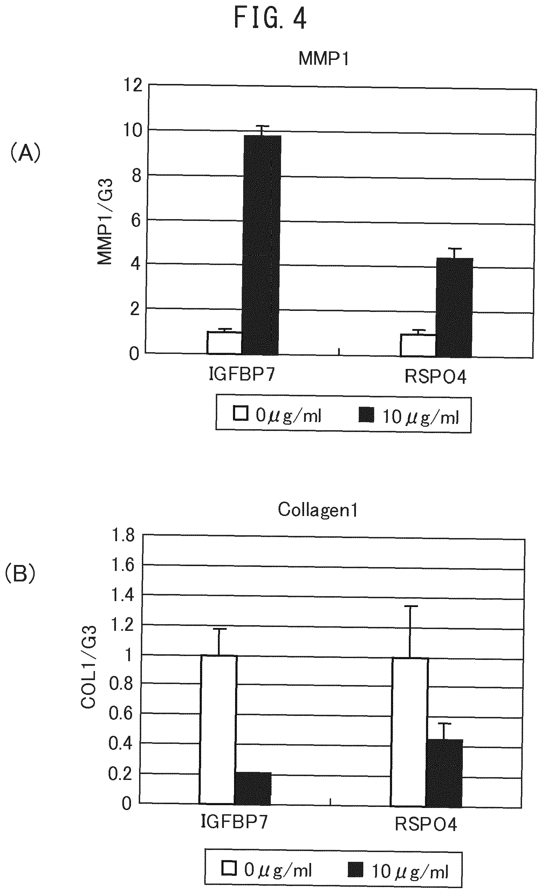

Proteins of the candidate aging factors (IGFBP 7 and RSPO 4) (purchased from Serotec, R&D SYSTEMS) were added to a medium for culturing young fibroblasts at concentrations of 0 µg/ml and 10 µg/ml, cultured for 2 days, and the cells were collected using Qiazole (Qiagen). The recovered cell lysate was examined for the expression levels of MMP1 and collagen in the same manner as described above for quantitative RT-PCR. The results are shown in FIG. 4. IGFBP7 and RSPO4 reduced the expression of collagen and increased the expression of MMP1. This indicated that IGFBP7 and RSPO4 also act as aging factors.

### Example 2: Activation of normal cells using mesenchymal stem cells

### Cell culture

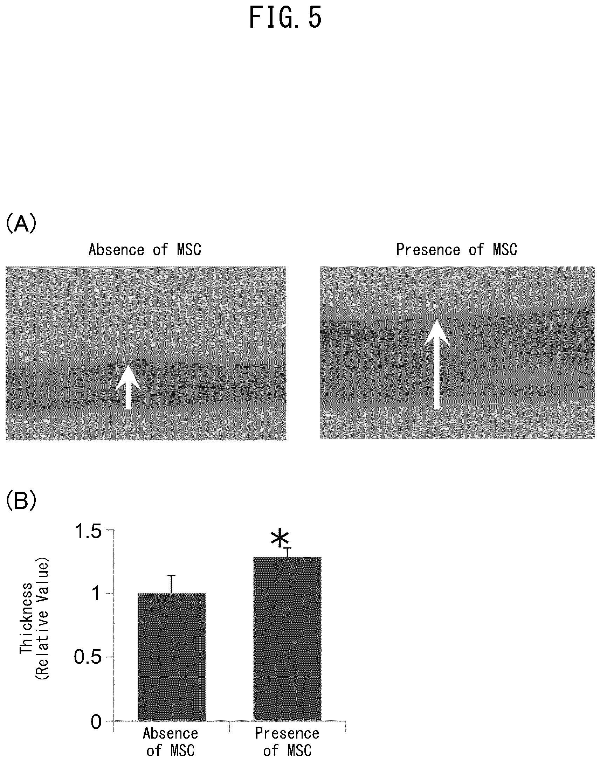

Dermal fibroblasts were purchased from Lonza and seeded in the upper layer of a six-well Transwell system (Falcon; Franklin Lakes, NJ) filled with Dulbecco's Modified Eagle Medium supplemented with 10% FBS to a density of 1250 cells/cm². Mesenchymal stem cells (MSC) were purchased from Lonza and cultured in a Gibco medium. MSCs in the sub-constructed state were seeded in the wells of the lower layer of the Transwell system. After 24 hours, the medium was changed to DMEM containing 10% FBS and 250 uM ascorbic acid, and co-culture was started. Seven days later, the upper layer was fixed with paraformaldehyde and stained with HE (FIG. 5A). The thickness of the upper layer was measured (FIG. 5B). As a control group, fibroblasts alone were cultured without MSC. In the presence of MSC, the dermal fibroblasts were activated and the degree of stratification of the cells increased (Student's t-test: P < 0.05).

### Example 3: Collagen gel maturation with mesenchymal stem cells

### Cell culture

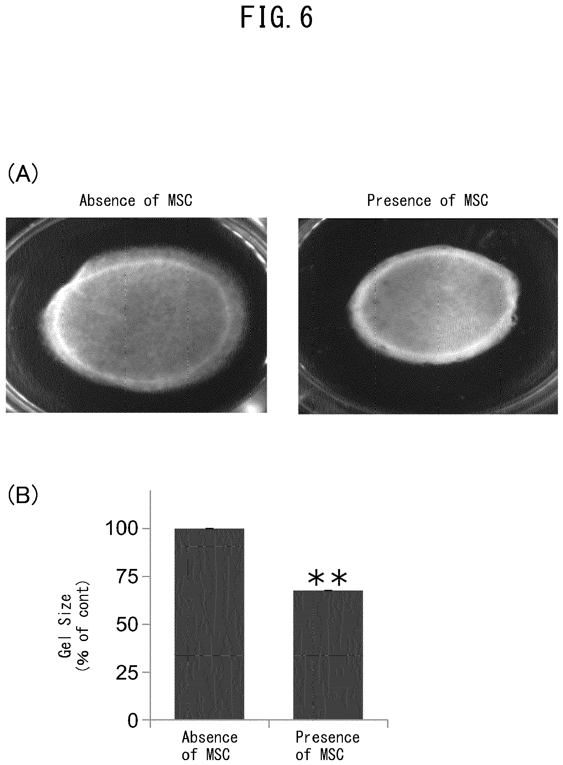

A fibroblast-retaining collagen gel obtained from Lonza was placed in the upper layer of a six-well Transwell system (Falcon; Franklin Lakes, NJ) filled with Dulbecco's Modified Eagle Medium supplemented with 10% FBS. Mesenchymal stem cells (MSC) were purchased from Lonza and cultured in a MessenPro medium. The MSCs in a sub-constructed state were seeded in the wells of the lower layer of the Transwell system. After 24 hours, the medium was changed to DMEM containing 10% FBS and 250 uM ascorbic acid, and co-culture was started. Two days later, a top-down photograph was taken, and the surface area of the collagen gel was compared. As a control group, collagen was cultured without MSC. The results are shown in FIG. 6 (Student's t-test: P < 0.05). It was found that the collagen gel contracted in the presence of MSC. This contraction is thought to be a result of increased elasticity as the collagen matures.

### Example 4: Aging factor suppression due with mesenchymal stem cells

### Cell Culture

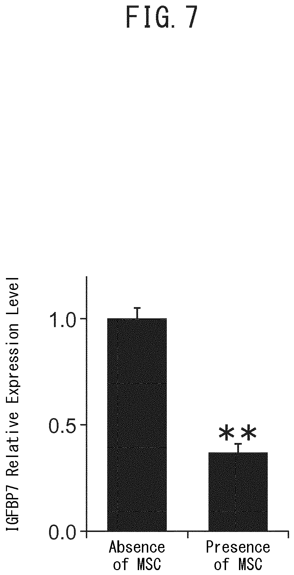

Senescent fibroblasts were seeded in the upper layer of a six-well Transwell system (Falcon; Franklin Lakes, NJ) filled with Dulbecco's Modified Eagle Medium supplemented with 10% FBS to a density of 1250 cells/cm². Mesenchymal stem cells (MSC) were purchased from Lonza and cultured in a MessenPro medium. The MSCs in a sub-constructed state were seeded in the wells of the lower layer of the Transwell system. After 24 hours, the medium was changed to DMEM containing 10% FBS and 250 µM ascorbic acid, and co-culture was started. Two days later, the cells were collected using Qiazole (Qiagen). The collected cell lysate was examined for the gene expression of the aging factor (IGFBP 7) using the same method as RT-PCR described above. The results are shown in FIG. 7. As a control group, fibroblasts alone were cultured without MSC. In the presence of MSC, the gene expression of IGFBP7 was significantly reduced (Student's t-test: P < 0.05).

### Example 5: Determination of mesenchymal stem cell attraction action

### Mesenchymal stem cell attraction test

Mesenchymal stem cells (Lonza) were cultured in a flask containing a MessenPro RS medium (DMEM; GIBCO/BRL (Carlsbad, CA)) at 37 °C and 5% CO₂ humidification. At subconfluence, the cells were harvested using Accutase (PAA (Linz, Austria)) and labeled using 2 µM PKH67 (Sigma-Aldrich (St. Louis, MO)) according to the manufacturer's instructions. 3 × 10⁴ labeled cells/well were seeded in the upper well of a 24-well Fluoroblok plate (Becton Dickinson (Mountain View, CA)). After 6 hours of incubation, the medium was replaced with a MessenPro RS medium without supplement (DMEM; GIBCO/BRL (Carlsbad, Calif.)). Incubation was continued for 12 hours and test substances were added to the lower wells of Fluoroblok at a concentration of 0.1%. After 8 hours, the lower side of the Fluorobolok membrane was photographed through a microscope equipped with a fluorescent filter and the cells attracted through the membrane were counted as a fluorescent area larger than the pore size using ImageJ. The attractiveness of adipose-derived stem cells was evaluated by comparing the fluorescence value in the control well with the fluorescence value in the test well.

As test extracts, a large number of cosmetic ingredients were subjected to screening. From thereamong, it was shown that iris extract (Koei Kogyo), olive leaf extract (Maruzen Pharmaceutical), and chamomile extract (Maruzen Pharmaceutical) have excellent mesenchymal stem cell inducing activity. Wells supplemented with iris extract, olive leaf extract, and chamomile extract showed significantly higher fluorescence compared to control wells containing extraction solvent only (Table 1, FIGS. 5A and B, Student's t-test: P < 0.05). In the case of iris extract, olive leaf extract, and chamomile extract, the extraction solvent was 50% butylene glycol. Instead of the test extract, only this solvent was added to the control well.

[SEQUENCE LISTING]

## Claims

1. A mesenchymal stem cell attracting agent, comprising at least one selected from the group consisting of iris extract, olive leaf extract, and chamomile extract.

2. An aging-factor production inhibiting agent comprising the attracting agent according to claim 1.

3. A dermal regeneration promoting agent comprising the attracting agent according to claim 1.
